(19) Europäisches Patentamt
European Patent Office
Office européen des brevets

(11) Veröffentlichungsnummer : **0 217 280 B1**

(12) # EUROPÄISCHE PATENTSCHRIFT

(45) Veröffentlichungstag der Patentschrift : **03.01.90**

(51) Int. Cl.$^5$ : **C 07 C 45/37**, C 07 C 47/19, B 01 J 35/08

(21) Anmeldenummer : **86113120.9**

(22) Anmeldetag : **24.09.86**

(54) **Verfahren zur Herstellung von Hydroxycarbonyl-Verbindungen aus 1,2-Diolen.**

(30) Priorität : **04.10.85 DE 3535483**

(43) Veröffentlichungstag der Anmeldung : **08.04.87 Patentblatt 87/15**

(45) Bekanntmachung des Hinweises auf die Patenterteilung : **03.01.90 Patentblatt 90/01**

(84) Benannte Vertragsstaaten : **BE CH DE FR GB IT LI NL**

(56) Entgegenhaltungen :
**EP-A- 0 002 781**
**EP-A- 0 125 515**
**DE-B- 1 278 411**
**GB-A- 1 234 766**
**GB-A- 2 069 366**

(73) Patentinhaber : **HOECHST AKTIENGESELLSCHAFT
Postfach 80 03 20
D-6230 Frankfurt am Main 80 (DE)**

(72) Erfinder : **Driscoll, Robert Kenneth, Dr.
Heilmann-Strasse 43
D-6000 frankfurt am Main 50 (DE)**
Erfinder : **Leupold, Ernst Ingo, Dr.
Am Zäunefeld 15
D-6392 Neu-Anspach (DE)**
Erfinder : **Schütz, Joachim, Dr.
Kurhausstrasse 57A
D-6238 Hofheim am Taunus (DE)**

**EP 0 217 280 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von Hydroxycarbonyl-Verbindungen durch Oxydehydrierung von 1.2-Diolen der Formel $R-CH(OH)CH_2OH$ mit R=H, Methyl, Ethyl, n-Propyl oder i-Propyl in der Gasphase an Silber-, Kupfer- oder Gold-Metallkatalysatoren.

Ein solches Verfahren ist bereits aus der GB-PS 1 234 766 bekannt. Die Katalysatoren werden dabei vorzugsweise als ungeträgerte Metalle eingesetzt. Als zweckmäßige Form der Katalysatoren werden Metallgazespiralen empfohlen. Die Ausführungsbeispiele betreffen sämtlich die Herstellung von Glykoladehyd aus 1.2-Ethandiol. Die Verweilzeiten liegen dabei zwischen 2 und 9 Sekunden und die Umsätze bei 70 %. Die Glykolaldehyd-Raum-Zeit-Ausbeuten sind kleiner als 12 g/l.h und damit technisch uninteressant. Neben Glykolaldehyd entstehen immer beträchtliche Mengen an Glyoxal. Für dessen Abtrennung wird eine Behandlung mit Bisulfit empfohlen ; .diese Methode ist jedoch nicht für ein technisches Großverfahren geeignet. Die genannten schlechten Umsätze führen dazu, daß viel 1.2-Ethandiol im Reaktoraustrag vorhanden ist. Es ist bekannt, daß Diole sehr leicht z. B. mit Glyoxal und Glykolaldehyd in der kondensierten Phase reagieren. Es entstehen dabei stabile Dioxolane, die schwierig zu spalten sind. Diese Reaktionen führen also zu Ausbeute-Verlusten und schwierigen Trennungsproblemen.

Es bestand daher die Aufgabe, einen metallischen Katalysator zu entwickeln, der in hohen Umsätzen und hohen Selektivitäten zu Hydroxycarbonyl-Verbindungen führt, um unerwünschte Folgereaktionen in der kondensierten Phase zu vermeiden und um die Gewinnung von reiner Hydroxycarbonyl-Verbindung zu erleichtern. Weiterhin soll der Katalysator hohe Raum-Zeit-Ausbeuten liefern.

Gegenstand der vorliegenden Erfindung ist daher ein Verfahren zur Herstellung von Hydroxycarbonyl-Verbindungen durch Oxydehydrierung von 1.2-Diolen der Formel $R-CH(OH)CH_2OH$ mit R=H, Methyl, Ethyl, n-Propyl oder i-Propyl in der Gasphase an Silber-, Kupfer- oder Gold-Metallkatalysatoren, dadurch gekennzeichnet, daß die Katalysatoren Metall-Hohlkugeln sind.

Die erfindungsgemäßen Katalysatoren liefern Glykolaldehyd-Raum-Zeit-Ausbeuten von mehr als 490 g/l·h. In der GB-PS 1 234 766 wurden Umsätze um 70 % bei Verweilzeiten von über 2 Sekunden und Belastungen bis etwa 41 g/l·h Diol an Silber- oder Kupfergazespiralen erzielt. Es ist daher überraschend, daß ähnliche Temperaturen und Gasphasezusammensetzungen mit dem erfindungsgemäßen Verfahren Umsätze von über 90 % bei Verweilzeiten von nur 0,05 Sekunden und Belastungen von 975 g/l·h erzielt werden können.

Die erfindungsgemäß eingesetzten Hohlkugeln können mit verschiedenen Durchmessern und Wanddicken, z. B. durch galvanische Methoden hergestellt werden. Dabei wird z. B. eine Kupfer-, Silber- oder Goldschicht auf Polystyrolkugeln geeigneter Größe galvanisch aufgebracht. Anschließend wird das Polystyrol durch Erhitzen entfernt, so daß eine hohle Metallkugel übrigbleibt. Das dabei entweichende Gas erzeugt kleine Löcher in der Metallkugel. Solche Kugeln sind im Handel erhältlich. Der Kugeldurchmesser beträgt vorzugsweise 0,5 mm bis 8 mm. Besonders bevorzugt ist ein Kugeldurchmesser von 1,0 mm bis 4 mm.

Die Wanddicke der Hohlkugeln wird so klein gewählt, daß ein hohes Verhältnis geometrische Oberfläche : Gewicht gewährleistet ist, um damit eine wirtschaftlich optimale Nutzung des Metalls zu erzielen. Die Kugeln am Boden einer Schüttung müssen eine entsprechende Wandstärke aufweisen, um dem Gewicht der Schüttung standzuhalten. Auch durch die Aufteilung der Schüttung in mehrere, aufeinandergestapelte Teilschüttungen unter Einsatz von gestützten Siebböden im Reaktorrohr kann dieses Problem gelöst werden.

Die Wanddicke der Hohlkugeln liegt vorzugsweise zwischen $10^{-3}$ mm und 0,5 mm, insbesondere zwischen $10^{-2}$ mm und $5.10^{-2}$ mm.

Die BET-Oberfläche der Kugeln beträgt vorzugsweise weniger als 5 $m^2/g$. Besonders bevorzugt sind jedoch BET-Oberflächen unterhalb 1 $m^2/g$.

Bei dem erfindungsgemäßen Verfahren können Hohlkugelschüttungen verwendet werden, deren Kugeln aus nur einem Metall bestehen (z. B. nur Silber) oder auch aus mehreren Metallen, z. B. Ag-Kugeln und Au-Kugeln. Die Kugeln aus verschiedenen Metallen können entweder in aufeinander gestapelten Teilschüttungen oder in miteinander gemischten Gesamtschüttungen angeordnet sein. Bevorzugt ist jedoch die alleinige Verwendung von Kupfer-Hohlkugeln.

Bei dem erfindungsgemäßen Verfahren werden 1.2-Diole der allgemeinen Formel $R-CH(OH)CH_2OH$ in der Gasphase eingesetzt, wobei R=H, Methyl, Ethyl, n-Propyl oder i-Propyl bedeutet. Bevorzugt ist jedoch 1.2-Ethandiol. Die Oxydehydrierung erfolgt nach den Formeln

$$R\text{-}CH(OH)CH_2OH + 1/2\ O_2 = R\text{-}\underset{\underset{\displaystyle O}{\|}}{C}CH_2OH + H_2O \text{ und}$$

$$R\text{-}CH(OH)CH_2OH + 1/2\ O_2 = R\text{-}CH(OH)CHO + H_2O.$$

Es hat sich als vorteilhaft erwiesen, der Gasphase Wasserdampf zuzugeben, um die Abscheidung von Feststoffen auf dem Katalysator oder im Kondensationsteil hinter dem Reaktorausgang zu unterbinden. Das Molverhältnis Wasser : 1.2-Diol liegt vorzugsweise zwischen 0,2 : 1 und 10 : 1, besonders bevorzugt

zwischen 1 : 1 und 4 : 1.

Das jeweilige gasförmige Diol und Wasser werden zusammen mit Sauerstoff oder einem sauerstoffhaltigen Gas, wie Luft, über den Katalysator geleitet. Vorzugsweise verdünnt man mit einem Trägergas, wie Stickstoff oder Edelgasen.

Das Molverhältnis Trägergas : 1.2-Diol liegt vorzugsweise zwischen 0,1 : 1 und 500 : 1.

Die Glykolaldehyd-Selektivität hängt vom Molverhältnis $O_2$ : Diol ab. Werte unter 0,5 : 1 (stöchiometrisches Verhältnis) können zwar verwendet werden, jedoch sind dann die Umsätze entsprechend gering. Die unerwünschte $CO_2$-Bildung steigt mit zunehmendem $O_2$ : Diol-Verhältnis an, so daß schon bei Molverhältnissen über 2,5 : 1 das Hauptprodukt normalerweise $CO_2$ ist. Das Molverhältnis $O_2$ : Diol liegt daher vorzugsweise zwischen 0,5 : 1 und 2,5 : 1, besonders bevorzugt zwischen 0,5 : 1 und 1,5 : 1 und insbesondere zwischen 0,55 : 1 und 0,75 : 1.

Die erfindungsgemäße Oxydehydrierung wird im allgemeinen bei Temperaturen zwischen 100° und 600 °C, vorzugsweise zwischen 250° und 450 °C durchgeführt.

Die Verweilzeit liegt vorzugsweise zwischen 0,01 und 10,0 Sekunden, insbesondere jedoch zwischen 0,01 und 1 Sekunde.

Die Reaktion wird bevorzugt bei Normaldruck durchgeführt, jedoch können auch verminderte oder erhöhte Drucke angewendet werden, d. h. etwa 0,01 bis 100 bar.

Im einzelnen geht man so vor, daß das 1.2-Diol, Wasser und Sauerstoff oder sauerstoffhaltiges Gas, sowie ggf. das Trägergas aus Dosierungsvorrichtungen in eine Verdampfungszone und die entstandene Gasmischung dann durch ein von außen beheiztes und mit dem Katalysator gefülltes Reaktionsrohr geleitet wird. Es hat sich dabei als vorteilhaft erwiesen, den Sauerstoff oder das sauerstoffhaltige Gas, sowie das Trägergas vor der Einleitung in die Verdampfungszone auf die Reaktionstemperatur aufzuheizen.

Das erfindungsgemäße Verfahren liefert hohe Selektivitäten an Hydroxycarbonyl-Verbindung bei hohen Umsätzen. Die reinen Hydroxycarbonyl-Verbindungen können mit verschiedenen Methoden aus dem Reaktionsgemisch isoliert werden: z. B. durch Acetalisierung der im Reaktoraustrag enthaltenen Carbonylverbindungen mit einem Alkohol, destillative Trennung der entstandenen Acetale und anschließend hydrolytische Spaltung der Hydroxycarbonyl-Acetale.

Glykolaldehyd findet Verwendung in der Herstellung von Polymeren, die freie OH-Gruppen enthalten. Es ist außerdem ein nützliches Zwischenprodukt in der Herstellung von Estern und Aminoalkoholen.

Die nachfolgenden Beispiele sollen die Erfindung näher erläutern.

## Beispiel 1

Der Katalysator, der aus Silberhohlkugeln mit 1,6 mm Durchmesser und $3{,}2 \cdot 10^{-2}$ mm Wanddicke bestand, wurde in einen senkrecht angeordneten Stahlreaktor von 200 mm Länge und 16 mm Innendurchmesser eingefüllt. Der Reaktor wurde von außen beheizt, und die Temperatur im Inneren wurde mit Hilfe eines Thermoelementes gemessen.

Eine wäßrige Lösung, die 50 Gew.% 1.2-Ethandiol enthielt, wurde mit einer Kolbenspritze über eine Verdampfungszone von oben in den senkrecht angeordneten Reaktor dosiert. Dabei wurden der Verdampfungszone gleichzeitig Stickstoff und Sauerstoff zugeführt, die beide vorher auf die jeweilige Reaktionstemperatur aufgeheizt wurden.

Nach einer Anlaufzeit von 1 Stunde zur Einstellung konstanter Betriebsbedingungen wurde der Versuch über 3 Stunden fortgeführt.

Der Reaktoraustrag wurde direkt hinter dem Reaktor in einer Kühlfalle kondensiert und mit gaschromatographischen Methoden analysiert.

Die Zusammensetzung der Gasphasen, Reaktionsbedingungen und die Produktzusammensetzung für 4 verschiedene Versuche mit diesem Katalysator sind in der Tabelle 1 wiedergegeben.

Beim Versuch 2 wurde mit 91,4 % Umsatz eine Raum-Zeit-Ausbeute von 499 g Glykolaldehyd pro Liter Silberhohlkugelkatalysator und Stunde erzielt. Die Ergebnisse von Versuch 1 bei 350 °C Reaktionstemperatur und Versuch 3 bei 370 °C sind sehr ähnlich. Versuch 4 demonstriert, daß gute Glykolaldehyd-Selektivitäten auch bei längeren Verweilzeiten erzielt werden können.

## Beispiel 2

Die Versuchsanordung war wie in Beispiel 1 beschrieben. Der Katalysator bestand aus Kupferhohlkugeln und hatte ein Schüttvolumen von 6 ml. Die Kugeln hatten einen Durchmesser von 1,6 mm und eine Wanddicke von $3{,}2 \cdot 10^{-2}$ mm. Ergebnisse mit diesem Katalysator sind in den Versuchen 5, 6 und 7 der Tabelle 1 wiedergegeben.

Die Versuche 5 und 1 bzw. 6 und 2 bieten einen direkten Vergleich zwischen Kupfer- und Silberhohlkugeln. Die Kupferkugeln ergeben eine deutlich höhere Selektivität zu Glykolaldehyd und insbesondere beträchtlich weniger $CO_2$ unter identischen Reaktionsbedingungen. Weniger $CO_2$ bedeutet auch, daß weniger Reaktionswärme durch die Reaktorwand abgeführt werden muß. Die höhere Selektivität zu Glykolaldehyd erleichtert dessen Isolierung aus dem Reaktoraustrag und steigert die erzielbaren Raum-Zeit-Ausbeuten auf über 600 g/l·h.

| Versuch | Katalysator: Typ und Schüttvolumen | Molverhältnis 1.2-Ethandiol:$O_2$:$N_2$:$H_2O$ | Gesamter Gasdurchsatz Mol/Stunde | Maximale Reaktions-Temperatur (°C) | Verweilzeit (s) |
|---|---|---|---|---|---|
| 1 | Silber 6 ml | 1,0 : 0,6 : 191,8 : 3,4 | 9,27 | 350° | 0,049 |
| 2 | " " | 1,0 : 0,6 : 96,8 : 3,4 | 9,62 | 350° | 0,047 |
| 3 | " " | 1,0 : 0,6 : 191,7 : 3,4 | 9,27 | 370° | 0,047 |
| 4 | Silber 25 ml | 1,0 : 0,6 : 95,7 : 3,4 | 4,75 | 360° | 0,39 |
| 5 | Kupfer 6 ml | 1,0 : 0,6 : 191,8 : 3,4 | 9,27 | 350° | 0,049 |
| 6 | " " | 1,0 : 0,6 : 96,8 : 3,4 | 9,62 | 350° | 0,047 |
| 7 | " " | 1,0 : 1,5 : 195,2 : 3,4 | 9,47 | 350° | 0,048 |

| Versuch | Umsatz % | Selektivität Glykolaldehyd % | Selektivität Glyoxal % | Selektivität $CO_2$ % | Raum-Zeit-Ausbeute an Glykolaldehyd g/l·h |
|---|---|---|---|---|---|
| 1 | 96,2 | 49,6 | 30,6 | 17,0 | 224,7 |
| 2 | 91,4 | 57,9 | 24,2 | 16,0 | 499,0 |
| 3 | 97,7 | 50,4 | 31,8 | 14,5 | 231,7 |
| 4 | 93,2 | 59,5 | 21,2 | 16,0 | 62,7 |
| 5 | 94,9 | 75,6 | 14,7 | 4,5 | 333,7 |
| 6 | 90,8 | 73,4 | 17,6 | 5,0 | 628,6 |
| 7 | 97,7 | 51,3 | 34,2 | 13,0 | 236,0 |

## Patentansprüche

1. Verfahren zu Herstellung von Hydroxycarbonyl-Verbindungen durch Oxydehydrierung von 1.2-Diolen der Formel R-CH(OH)$CH_2$OH mit R=H, Methyl, Ethyl, n-Propyl oder i-Propyl in der Gasphase an Silber-, Kupfer- oder Gold-Metallkatalysatoren, dadurch gekennzeichnet, daß die Katalysatoren Metall-Hohlkugeln sind.

2. Verfahren nach Anspruch 1, dadurch gekennzeichnet, daß die Metall-Hohlkugeln aus Kupfer bestehen.

3. Verfahren nach Anspruch 1 oder 2, dadurch gekennzeichnet, daß die Metall-Hohlkugeln eine BET-Oberfläche von weniger als 5 $m^2$ $g^{-1}$ besitzen.

4. Verfahren nach einem der Ansprüche 1 bis 3, dadurch gekennzeichnet, daß die Metall-Hohlkugeln eine Wanddicke zwischen $10^{-3}$ mm und 0,5 mm besitzen.

5. Verfahren nach einem der Ansprüche 1 bis 3, dadurch gekennzeichnet, daß die Metall-Hohlkugeln eine Wanddicke zwischen $10^{-2}$ mm und $5 \cdot 10^{-2}$ mm besitzen.

6. Verfahren nach einem der Ansprüche 1 bis 5, dadurch gekennzeichnet, daß die Verweilzeit zwischen 0,01 und 10 Sekunden liegt.

7. Verfahren nach einem der Ansprüche 1 bis 5, dadurch gekennzeichnet, daß die Verweilzeit zwischen 0,01 und 1 Sekunde liegt.

8. Verfahren nach einem der Ansprüche 1 bis 7, dadurch gekennzeichnet, daß das Molverhältnis Sauerstoff : 1.2-Diol zwischen 0,5 : 1 und 1,5 : 1 liegt.

9. Verfahren nach einem der Ansprüche 1 bis 8, dadurch gekennzeichnet, daß die Reaktionstemperatur zwischen 250° und 450 °C liegt.

## Claims

1. A process for the preparation of hydroxycarbonyl compounds by oxydehydrogenation of 1,2-diols of the formula R-CH(OH)$CH_2$OH, where R=H, methyl, ethyl, n-propyl or i-propyl, in the gas phase on silver, copper or gold metal catalysts, wherein the catalysts are hollow metal spheres.

2. The process as claimed in claim 1, wherein the hollow metal spheres comprise copper.

3. The process as claimed in claim 1 or 2, wherein the hollow metal spheres have a BET surface area of less than 5 $m^2$ $g^{-1}$.

4. The process as claimed in any one of claims 1 to 3, wherein the hollow metal spheres have a wall thickness between $10^{-3}$ mm and 0.5 mm.

5. The process as claimed in any one of claims 1 to 3, wherein the hollow metal spheres have a wall thickness between $10^{-2}$ mm and $5.10^{-2}$ mm.

6. The process as claimed in any one of claims 1 to 5, wherein the residence time is between 0.01 and 10 sec.

7. The process as claimed in any one of claims 1 to 5, wherein the residence time is between 0.01 and 1 sec.

8. The process as claimed in any one of claims 1 to 7, wherein the oxygen : 1,2-diol molar ratio is between 0.5.: 1 and 1.5 : 1.

9. The process as claimed in any one of claims 1 to 8, wherein the reaction temperature is between 250° and 450 °C.

## Revendications

1. Procédé pour préparer des composés hydroxycarbonyliques par oxydéshydrogénation de diols-1,2 répondant à la formule :

$$R\text{-}CH(OH)CH_2OH$$

dans laquelle R représente H ou un radical méthyle, éthyle, n-propyle ou isopropyle, en phase gazeuse, en présence de catalyseurs métalliques en argent, en cuivre ou en or, procédé caractérisé en ce que les catalyseurs sont des billes métalliques creuses.

2. Procédé selon la revendication 1. caractérisé en ce que les billes métalliques creuses sont en cuivre.

3. Procédé selon l'une des revendications 1 et 2, caractérisé en ce que les billes métalliques creuses ont une surface BET inférieure à 5 $m^2$ $g^{-1}$.

4. Procédé selon l'une quelconque des revendications 1 à 3, caractérisé en ce que les billes métalliques creuses ont une épaisseur de paroi comprise entre $10^{-3}$ mm et 0,5 mm.

5. Procédé selon l'une quelconque des revendications 1 à 3, caractérisé en ce que les billes métalliques creuses ont une épaisseur de paroi comprise entre $10^{-2}$ mm et $5.10^{-2}$ mm.

6. Procédé selon l'une quelconque des revendications 1 à 5, caractérisé en ce que le temps de séjour est compris entre 0,01 et 10 secondes.

7. Procédé selon l'une quelconque des revendications 1 à 5, caractérisé en ce que le temps de séjour est compris entre 0,01 et 1 seconde.

8. Procédé selon l'une quelconque des revendications 1 à 7, caractérisé en ce que le rapport molaire entre l'oxygène et le diol-1,2 est compris entre 0,5 : 1 et 1,5 : 1.

9. Procédé selon l'une quelconque des revendications 1 à 8, caractérisé en ce que la température réactionnelle est comprise entre 250 et 450 °C.